# EUROPÄISCHE PATENTANMELDUNG

(11) **EP 4 265 266 A1**
(43) Veröffentlichungstag der Anmeldung: **25.10.2023**
(21) Anmeldenummer: 23162492.5
(22) Anmeldetag: 16.03.2023
(51) Int. Cl.: A61K 36/43, A61K 35/644, A61P 35/00

(54) **KIT OF PARTS ZUR THERAPIE VON MEDULLOBLASTOMEN UND DIFFUSEN ASTROZYTOMEN MITTELS ALKOHOLISCHER UND/ODER W SSRIGER EXTRAKTE VERSCHIEDENER CUSCUTA SPP**

(71) Anmelder: Patentpool Target GmbH, 80331 München (DE)
(72) Erfinder: Burda, Renate, 82054 Sauerlach (DE); M rth-Kretschmer, Jenny, 21244 Buchholz in der Nordheide (DE); Sand, Elisabeth, 91567 Rauenzell/Herrieden (DE); Dirk, Weickmann, 91567 Rauenzell/Herrieden (DE)
(74) Vertreter: Reich, Jochen

(57) **Zusammenfassung**

Die Erfindung betrifft eine pharmazeutische Zusammensetzung zur Behandlung eines Medulloblastomes und/oder eines diffusen Astrozytomes, enthaltend in einer pharmazeutisch wirksamen Menge eines wässrigen oder alkoholischen Auszuges aus Pflanzen der Gattung Cuscuta sowie die Verwendung der pharmazeutischen Zusammensetzung zur Behandlung eines Medulloblastomes und/oder eines Astrozytomes, ein Verfahren zur Herstellung der pharmazeutischen Zusammensetzung sowie ein Kit of parts enthaltend die pharmazeutische Zusammensetzung.

## Beschreibung

Die vorliegende Erfindung betrifft eine pharmazeutische Zusammensetzung, die Verwendung der pharmazeutischen Zusammensetzung zur Behandlung eines Medulloblastomes und/oder eines diffusen Astrozytomes, ein Verfahren zur Herstellung der pharmazeutischen Zusammensetzung sowie ein Kit of parts, enthaltend die pharmazeutische Zusammensetzung.

In Deutschland treten pro Jahr mindestens 10000 Fälle bösartiger Hirntumore auf. Dabei handelt es sich um Astrozytome mit Malignitätsgrad II und III, sowie Glioblastome des Grades IV und Glioblastoma multiforme aber auch um Medulloblastome, welche vor allem bei Kindern auftreten. Ein Hirntumor wird auch heute noch meist als Nebenbefund diagnostiziert. Regelmässig treten aber auch Blutungen im Schädel- und Hirnbereich der Patienten auf, die mit Bewusstlosigkeit und/oder starken, teilweise sehr starken Schmerzen einhergehen. Ein Klinikaufenthalt ist somit dringend notwendig.

Die Behandlung von Hirntumoren gestaltet sich als äußerst schwierig. Insbesondere bei Auftreten von Glioblastomen sind die Prognosen für den Patienten in der Regel als negativ anzusehen. Durch eine operative Tumorentfernung kann die Überlebenszeit um einige Monate verlängert werden und die Symptome gelindert werden. Eine dauerhafte Heilung ist jedoch höchst selten und bei Glioblastomen aus schulmedizinischer Sicht aktuell noch nahezu unmöglich.

Derzeit werden Tumore, als die gefährlichsten und gefürchtetsten Krankheiten unserer Zeit angesehen. Sie werden auf eine sehr radikale und den Patienten wenig schonende Weise bekämpft. Als einfache kennzeichnende Schlagworte können hier gelten: Stahl, Strahl und Chemotherapie Das bedeutet einmal, dass Tumore, falls einigermaßen erreichbar, im Prinzip operativ mit dem Skalpell herausgeschnitten, durch eine breitgefächerte Bestrahlung zerstört, oder über eine sogenannte Chemotherapie mit, auch gesunde Zellen angreifenden, aggressiven Chemotherapeutika/Zytostatika zerstört werden. Sowohl bei normalen Behandlungen mit dem Skalpell als auch mit ionisierender Strahlung ist eine räumliche Begrenzung des Operationsgebiets nicht möglich. Es werden zwangsläufig auch gesunde Körperzellen vernichtet. Die unerwünschten Nebenwirkungen der Chemotherapie sind allgemein bekannt, ebenso wie die Nachteile einer Radiotherapie. Impfstoffe stehen nicht zur Verfügung und sind aus zellbiologischer und biochemischer Sicht eher kontraproduktiv was den Einsatz bei Erkrankungen mit Zellentartungen betrifft.

Insbesondere die operative Entfernung von Hirntumoren hat jedoch weitere Nebenwirkungen. Häufig wird eine zweite Operation des Patienten notwendig. Bei der ersten Operation und Entfernung des Hirntumors wird eine große Narbenfläche erzeugt. Dort können sich neben gesunden Gliazellen auch kranke, d. h. maligne Gliazellen anlagern. Dies sind häufig Zellen, die aus dem Tumor stammen und Metastasen erzeugen. Dadurch ist ein diffuses Rezidiv vorprogrammiert. Dieses kann nicht mehr auf herkömmlichen Behandlungswegen behandelt werden. Daher ist dieses für einen Patienten oft letal.

Nach einer Operation erfolgt üblicherweise eine Strahlentherapie und/oder eine Chemotherapie. Bei der Chemotherapie wird häufig Cisplatin in verschiedenen Kombinationen verwendet. Seit Anfang 2002 wird auch das oral einzunehmende Temozolomid verwendet. Auch IMATINIB ist ein oral einzunehmendes Zytostatikum, das Anwendung findet. Neben den bekannten Nebenwirkungen wie Erbrechen, Unwohlsein, Depressionen, durch die Chemotherapie hervorgerufenen Krebs und Nervenleiden besteht bei den oben genannten Mitteln das Problem, dass sie zur Behandlung des Hirntumors die Blut-Hirn-Schranke überwinden müssen. Die Wirkstoffe durchschreiten die Blut-Hirn-Schranke üblicherweise lediglich mit 0,4 bis 5%. Die Zusammensetzung und Funktionsweise der Blut-Hirn-Schranke sind bis heute nur in Ansätzen verstanden. Diese Barriere spielt beim Schutz des Gehirns vor schädlichen Stoffen eine Rolle, ermöglicht andererseits aber auch eine geregelte Energiezufuhr.

Unser Körper besteht aus einzelnen Organsystemen und Organen, die für ihre Funktion unterschiedliche aber konstante Bedingungen zum Beispiel an Nährstoffen, Hormonen oder Elektrolyten benötigen. Alle Organe sind durch den Blutkreislauf miteinander verbunden. Da im Blut alle Bestandteile für die Versorgung, aber auch für die Entschlackung des Körpers enthalten sind, müssen Filtersysteme dafür sorgen, dass für die einzelnen Organsysteme nur die benötigten Stoffe durchgelassen beziehungsweise teilweise zurückgehalten werden. In diesem Zusammenhang kennt man unter anderem die Blut-Gewebe-Schranke, auch als Blut-Parenchym-Schranke bezeichnet, die Blut-Leber-Schranke, die Blut-Liquor-Schranke, die Blut-Hirn-Schranke, die Liquor-Hirn-Schranke, die Blut-Nerven-Schranke, die Blut-Retina-Schranke und die Plazenta-Schranke.

Bei diesen Filtermechanismen wird durch einen so genannten Schrankeneffekt der Übertritt bestimmter Stoffe aus der Blutbahn in das jeweilige Organsystem verhindert oder eingeschränkt, wenn diese Organsysteme die Bestandteile nicht oder nur in geringerer Konzentration benötigen. Diese "Schranken" sind keine selbständigen Organe, sondern sie werden aus einer Vielzahl von Zellen und Zellzwischenräumen gebildet, die die Blutgase, die Nährstoffe und bestimmte Chemikalien durchlassen oder als Endothelporen Makromoleküle zurückhalten. Sie können auch als Lipidmembranen in der Gefäßwand hemmend auf den Durchtritt nicht lipidlöslicher Stoffe wirken oder eine selektive Wirkung aktiver Transportprozesse in den Kapillaren bewirken. Das Gehirn und das Nervengewebe werden durch zwei Filtersysteme geschützt, die Blut-Liquor-Schranke und die Blut-Hirn-Schranke.

Das Vorhandensein und die Funktion der so genannten Blut-Hirn-Schranke ist schon seit über 100 Jahren bekannt und von Paul Ehrlich bereits 1885 im Experiment nachgewiesen worden. Innerhalb des Zentralnervensystems sind die Räume zwischen den Neuronen fast völlig durch Gliazellen und ihre Ausläufer ausgefüllt. Der gesamte Stoffwechsel der Nervenzellen geht über diese Glia- oder Endothelzellen. Sie dienen zum Einbau der Nervenzellen und Nervenfasern und zu ihrer Ernährung und Isolation. Eine Form der Gliazellen sind die Astrocyten. Sie besitzen zahlreiche Fortsätze, mit denen sie sich an der Wand der Kapillaren befestigen und eine die Kapillaren allseitig umgebende, nahezu spaltenlose Endothelauskleidung bilden. Diese Endothelzellen sind durch Verbindungselemente, die "tight junctions", verknüpft und mit einer selektiven Stoffdurchlässigkeit ausgestattet, die nur Partikel mit einem Durchmesser kleiner 20 nm passieren lässt. Auf diese Weise geht der gesamte Stoffwechsel der Nervenzellen über dieses endotheliale Geflecht, das die im Blut vorhandenen Substanzen gleich einem biologischen Filter bei Bedarf durchlässt, aber für die Gehirnfunktion schädliche Substanzen vom Nervensystem fernhält. Dieses Endothelgeflecht und die Endothelzellen, die die Kapillaren als eine Basalmembran auskleiden, werden als Blut-Hirn-Schranke bezeichnet. Ungehindert durchgelassen werden Sauerstoff, Kohlendioxid, D-Glukose, D-Hexose, einige L-Aminosäuren und lipidlösliche Stoffe, die für die Versorgung des Gehirns notwendig sind. Ebenso werden Abbauprodukte ins Blut abgegeben. Eine gewisse Barriere stellen die Endfortsätze der Astrocyten für zahlreiche Stoffe wie bestimmte Hormone, nicht lipidlösliche, wasserlösliche und chemische Substanzen sowie Proteine dar und sichern dadurch die Aufrechterhaltung eines konstanten Milieus für die Neuronen des Nervensystems.

Das Zellgefüge der Astrozyten ist so angeordnet, dass es eine effektive Abschottung gegen höhermolekulare Substanzen und Organismen bildet. Es ist aber auch unter Normalbedingungen nicht völlig dicht, sodass einige Partikel immer diese Schranke durchdringen können. Bei Infektionen, Traumen, Entzündungen , Vergiftungen, Hypoxidosen, Fieber und im Bereich von Tumoren werden die engen Verbindungen, die tight junctions, zwischen den Endothelzellen durch die Schwellung der Astrocythen aufgedehnt und deutlich durchlässiger für andere Stoffe. Die Änderung der Lichtungsweite erfolgt durch Quellung und Entquellung der Endothelzellen. Auch die Basalmembran der Kapillaren ist keine geschlossene Schicht. Je nach der Dichte des Fasernetzes entstehen Poren in der Membran, die aktiv am Stoffaustausch beteiligt sind.

Schon lange vor der Möglichkeit einer Antibiotikabehandlung hat man die Durchlässigkeit der Blut-Hirn-Schranke durch künstliche Fiebererzeugung ähnlich dem Vorgang bei Infektionen gesteigert und zur Therapie der Syphilis des Zentralnervensystems und zur Schockbehandlung in der Psychiatrie ausgenutzt, indem man Medikamente damit direkt an das Gehirn herangeführt hat. Nach dem Ende der Einwirkung der die Blut-Hirn-Schranke beeinflussenden Bedingungen bildet sich die zeitweise Durchlässigkeit wieder zurück.

Für die Chemotherapie von Patienten mit Hirnmetastasen solider Tumoren wird derzeit Termozolomid ein lipophiles Alkylans, klinisch erforscht. Es überwindet die Blut-Hirn-Schranke und erhöht die Strahlensensibilität von Tumoren bei simultaner Radiotherapie.

Im Gegensatz hierzu wurde aber auch versucht, eine Krebstherapie auf subtilere Weise auf Basis von Naturstoffen zu ermöglichen.

Es werden hierzu, unter anderem, viele aus giftigen Lebewesen isolierte, stark wirksame Stoffe in therapeutischen Dosen als Arzneistoffe genutzt.

Die Nutzung dieser biogenen Gifte begann schon früh in der Geschichte der Menschheit. Zur gefahrlosen Anwendung dieser Gifte waren jedoch von Anfang an gewisse Grundkenntnisse über deren Behandlung und Wirksamkeit erforderlich. Die weiter durchgeführten Versuche, die Zusammensetzung des chemischen Aufbaus biogener Gifte zu entschlüsseln, führten später zur gezielten Suche bestimmter Wirkstoffe als eigentliche Verursacher beobachteter Wirkungen. Einen gewaltigen Aufschwung in der Trenntechnik, dem Weg zur Ermittlung von Wirkstoffen zur Bekämpfung von Krankheiten, machte die Entwicklung chromatographischer Verfahren in der Mitte des 20. Jahrhunderts möglich. Ausgehend von der Verteilung zwischen einer mobilen und einer stationären flüssigen Phase, von der Adsorption, den Molekülsiebeffekten, dem lonenaustausch, der Affinität (insbesondere von Proteinen) zu bestimmten chemischen Verbindungen (z.B. Enzymsubstraten) und der Beweglichkeit geladener Moleküle im elektrischen Feld, wurde eine Vielzahl neuer Trenntechniken entwickelt.

Gerade in neuerer Zeit wurden aus biogenen Giften (aus Pilzen, Bakterien, Pflanzen und Tieren), viele pharmazeutische Wirkstoffe isoliert und weiter entwickelt.

So ist aus der PCT/EP00/12902 ein pharmazeutischer Wirkstoff bekannt, bei dem gefunden wurde, dass Bestandteile der Spinnengifte von Spinnen der Familie Sicariidae zur Behandlung von Tumorerkrankungen verwendet werden können. Es werden hierbei in der Hauptsache ein Peptidtoxin aus dem Gift dieser Spinnenart, eine weitere, aus dem Gift gewonnene, antagonistisch wirkende Substanz und/oder eine Kombination dieser Bestandteile medizinisch genutzt. Vorgeschlagen wird die Verwendung von Peptidtoxinen aus beispielsweise der Gattung Sicarius zur Behandlung von Mammakarzinomen, Lungenkarzinomen, Adenokarzinomen, Leberkarzinomen sowie Melanomen. Die Behandelbarkeit von Hirntumoren wie Astrozytomen und Glioblastomen wird dagegen nicht erwähnt. Konsequenterweise wird auch das Problem der Überwindung der Blut-Hirn-Schranke nicht diskutiert.

Die in der PCT/EP00/12902 beschriebenen Wirkstoffe können zur Behandlung von Tumorerkrankungen sowie parallel bzw. unterstützend bei Tumoroperationen eingesetzt und Rest-Tumorgewebe zerstört werden. Bei der Therapie können genetisch veränderte Körperzellen (Tumorzellen) zerstört werden, da der betreffende Wirkstoff die veränderte Oberflächenstruktur solcher Zellen erkennt und komplikationsfrei abtötet. Das Gesamtgift dieser Spinnenarten, sozusagen ein Cocktail verschiedener Substanzen, ist auf Grund seiner bereits in geringen Dosen letalen Wirkung nicht pharmazeutisch einsetzbar.

Dieser bekannte Wirkstoff wirkt jedoch in vivo in keiner Kombination bei einem Hirntumor, insbesondere nicht bei einer besonderen Art von Hirntumor, nämlich einem diffusen Astrozytom und/oder einem Medulloblastom. Zudem ist es für eine erfolgreiche Therapie notwendig, dass dieser Wirkstoff zu großen Teilen die Blut-Hirn-Schranke überwindet.

Eine weitere Kombination von Giftstoffen ist in der PCT/EP2006/063281 offenbart. Die darin offenbarte Kombination verschiedener Giftstoffe neutralisiert sich jedoch teilweise gegenseitig, so dass sich die erwünschte tumorzellzerstörende Wirkung nur unzureichend einstellt.

Es hat sich nämlich überraschenderweise herausgestellt, dass die Peptidtoxine von Loxosceles die Peptidtoxine von Latrodectus abbauen, so dass es zu keiner signifikanten Durchdringung der Blut-Hirn-Schranke der Loxosceles-Peptidtoxine kommt. Auf einem Elektrophoresegel zeigten sich nach einer Mischung der Peptidtoxine von Latrodectus und Loxosceles keine distinkten Banden, sondern lediglich ein Schmier, verursacht durch die Bruchstücke des Peptidverdaus der Latrodectus-Peptidtoxine. Spinnen der Gattung Loxosceles gehören wie Spinnen der Gattung Sicarius zur Familie der Sicariidae. Aufgrund der engen Verwandtschaft beider Spinnengattungen war deshalb auch für eine Mischung aus Peptidtoxinen der Gattung Sicarius mit solchen der Gattung Latrodectus keine Wirkung zu erwarten.

Es ist deshalb eine Aufgabe der vorliegenden Erfindung, eine Zusammensetzung bereitzustellen, die unter möglichst guter und hochprozentiger Überwindung der Blut-Hirn-Schranke eine komplikationslose Abtötung von kanzerösen Körperzellen aus dem Bereich des Hirngewebes, speziell von Medulloblastomen und/oder diffusen Astrozytomen, bewirkt.

Eine andere Aufgabe der vorliegenden Erfindung ist es, ein Verfahren bereitzustellen, das die Herstellung einer pharmazeutischen Zusammensetzung zur Behandlung von Medulloblastomen und/oder diffusen Astrozytomen (Mb/d A) ermöglicht.

Es ist eine weitere Aufgabe der vorliegenden Erfindung, eine Kombination von Wirkstoffen bereitzustellen, die eine wirksame Behandlung von Mb/d A ermöglicht.

Diese Aufgaben werden durch die Merkmale der unabhängigen Ansprüche gelöst. Bevorzugte Weiterbildungen und Ausgestaltungen der Erfindung finden sich in den Unteransprüchen.

Ein erster Aspekt der vorliegenden Erfindung betrifft daher eine pharmazeutische Zusammensetzung zur Behandlung von Mb/d A, enthaltend in einer pharmazeutisch wirksamen Menge

### 1. a) wässriger oder alkoholischer Auszug aus Pflanzen der Gattung Cuscuta

Ein wässriger oder alkoholischer Auszug der Pflanzen der Gattung Cuscuta ist in der Lage Zellen eines Mb/d A zu zerstören.

### Erfindungsgemäße Zusammensetzung und Herstellung:

Wir fanden nun in verschiedenen Cuscuta spp., Cuscuta epithymum, Cuscuta epilinum, Cuscuta europaea (Ansbach), Cuscuta campestris, Cuscuta gronovii und Cuscuta sp. (Ital. Macchie), Substanzen, bzw. Substanzgemische, die bestimmte Hirntumorzellen (Mb/d A) in vitro und in vivo abtöten können. Bislang verwendet man Cuscuta-Samen in der TCM gegen verschiedene Erkrankungen. Wir fanden in unseren Forschungen heraus daß ein Methanol-Extrakt, bzw. ein wässriger Extrakt von den vorgenannten Cuscuta species bestimmte Hirntumorzellen abtöten kann.

Desweiteren fanden wir heraus, dass ein Ethanol-Extrakt der frischen Stängelbereiche It. Zeichnung 1 der Cuscuta epithymum ssp., der in Honig eingearbeitet wird Mb/d A-Zellen abtöten kann.

Somit eignet sich ein in Honig eingearbeiteter ethanolischer oder wässriger Extrakt zur Therapie von Mb/d A.

Weltweit sind etwa 200 Arten Cuscuta bekannt. Viele haben pharmazeutisch-medizinisch interessante Substanzen. Nur wenige Arten wurden bislang hinsichtlich ihrer therapeutischen Wirkung untersucht. Wir untersuchten Extrakte der folgenden Cuscutaarten und -unterarten hinsichtlich ihrer Hirntumorzellen (speziell Mb/d A) abtötenden Wirkung:
- Cuscuta epilinum
- Cuscuta epithymum
- Cuscuta europaea (Ansbach)
- Cuscuta europaea (Lyon)
- Cuscuta lupuliformis
- Cuscuta campestris
- Cuscuta gronovii
- Cuscuta reflexa
- Cuscuta chinensis
- Cuscuta sp. (Ital. Macchie)

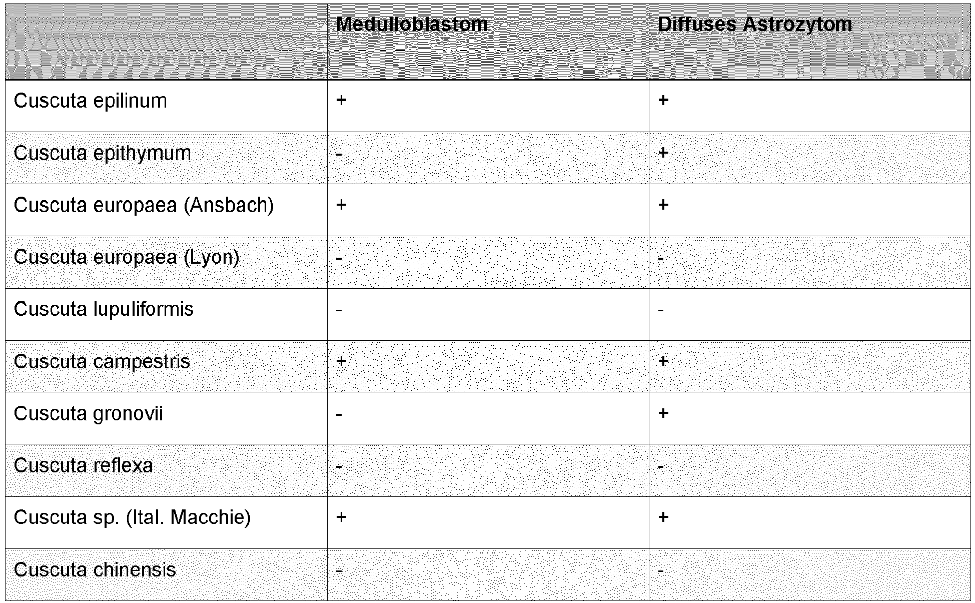

Nur Extrakte von Cuscuta epilinum, Cuscuta epithymum, Cuscuta europaea (Ansbach), Cuscuta campestris, Cuscuta gronovii und Cuscuta sp. (Ital. Macchie) zeigten einen nachweislichen erfindungsgemässen Effekt gegen Hirntumorzellen (Medulloblastom und/oder diffuses Astrozytom-Zellen.

Insbesondere bevorzugt bei der Zusammensetzung gemäß der vorliegenden Erfindung sind die Arten:
- Cuscuta epilinum, vom Aussterben bedrohte Art die ursprünglich zwischen Iran und Zentralasien, sowie in Europa verbreitet war. Heute gibt es noch eingeschleppte Vorkommen in Nordamerika.
- Cuscuta epithymum, weltweit verbreitet aber überall stark im Rückgang begriffen
- Cuscuta europaea, von den gemässigten Zonen Eurasiens bis ins nördliche Indien verbreitet
- Cuscuta europaea (Ansbach), im Landkreis Ansbach an der Altmühl zu finden
- Cuscuta campestris, ursprünglich in Nordamerika, in der Karibik und im westlichen Südamerika weit verbreitet
- Cuscuta gronovii, ursprünglich in Nordamerika von Kanada bis in die U.S.A. vorkommend. Auch auf Hispaniola zu finden.
- Cuscuta sp. (Ital. Macchie), in der Macchie zwischen Venedig und Istrien zerstreut zu finden

Die erfindungsgemäße Zusammensetzung wird gemäß einem Aspekt der vorliegenden Erfindung auf die folgende Weise hergestellt:
a) Es wird eine bestimmte Menge Stängel, in eine bestimmte Menge 96% Ethanol, bzw. bevorzugt 100% Ethanol oder wahlweise in eine bestimmte Menge Aqua ad injectabilia eingebracht.
b) Der Extrakt aus a) wird mindestens 24 Stunden bei Temperaturen von bevorzugt 25 Grad Celsius stehen gelassen. Anschließend werden die Stängel abfiltriert.
c) Dieser Wirkextrakt aus b) wird in 150 mL verflüssigten Bioblütenhonig unter ständigen Rühren eingearbeitet. Wahlweise kann auch der pure Extrakt verwendet werden.

Kultur der erfindungsgemäß bevorzugten Cuscuta sp.zur optimalen Wirkstoffsynthese:
Damit Pflanzen, vor allem die für die erfindungsgemäße Verwendung benötigten Wirkstoffe in optimaler Menge produzieren ist es optimal die jeweiligen Wirtspflanzen in Pflanztöpfen mit hochwertigen Pflanzsubstraten zu kultivieren.

Mehr als 30 verschiedene Komponenten lassen sich im Ethanol-Extrakt der Cuscuta epithymum, Cuscuta epilinum, Cuscuta europaea (Ansbach), Cuscuta campestris, Cuscuta gronovii und Cuscuta sp. (Ital. Macchie), nachweisen. Die Quantität der Wirkstoffe in den Pflanzen kann durch die richtige Substratverwendung der Wirtspflanzen gesteigert werden. Eine Kultur in Pflanzgefäßen ist effizienter als eine solche direkt im Boden. Bei Pflanzung direkt im Boden werden unter ungünstigen Bedingungen viel Zuwenig der Wirkstoffe produziert um effektiv wirksame, für die Therapie verwendbare Extrakte zu erhalten. Für die Erfindungsgemäße Anwendung ist es wichtig, die frischen Stängel der Pflanzen (siehe Zeichnung 1) zu verwenden.

Wirkungen konnten wir beobachten bei folgenden Konzentrationen:

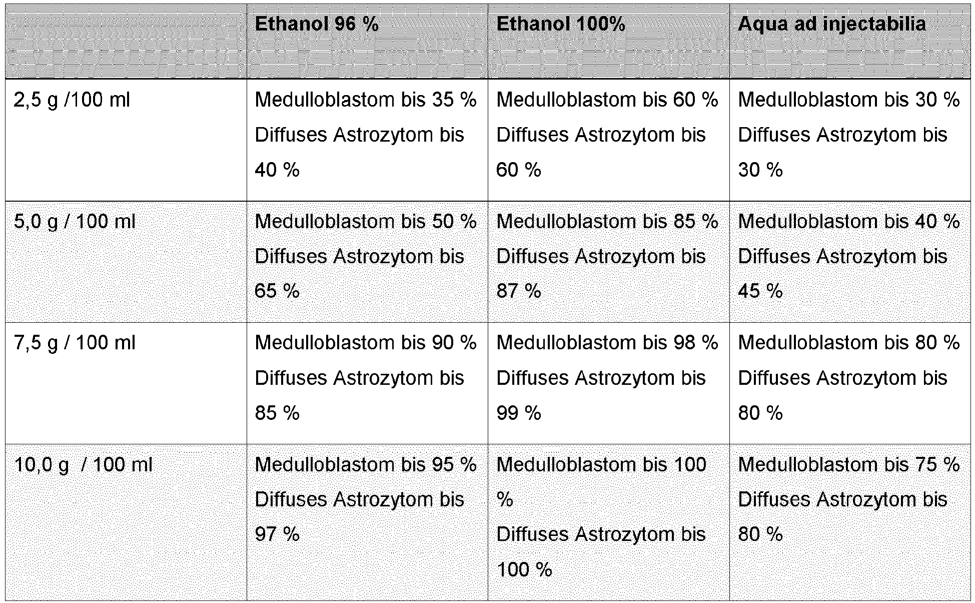

Somit sind die höheren Konzentrationen und die Auszüge in 100% Ethanol am effektivsten.

Ein weiterer Aspekt der vorliegenden Erfindung betrifft ein Kit of parts zur Behandlung von Gehirntumoren (Mb/d A) umfassend eine pharmazeutische Zusammensetzung wie oben beschrieben und einen Extrakt aus Weihrauch und/oder Bambus. Insbesondere bevorzugt wird hier der Extrakt aus Weihrauch- und/oder Bambusblättern verwendet. Die Hauptbestandteile des Weihrauchs sind Harze, in denen Boswelliasäuren und ätherische Öle enthalten sind. Neben den Boswelliasäuren, die zur Gruppe der pentacyclischen Triterpene gehören, findet man auch ein tetracyclisches Triterpen, die Tirucallsäure, in den Harzen.

Bevorzugt wird in der vorliegenden Erfindung das Präparat H15 Ayurmedica verwendet. Dabei handelt es sich um Tabletten, die jeweils 400 mg standardisierten Trockenextrakt aus Boswellia serata enthalten. Die Dosierung wird je nach Bedarf angepasst. Sie kann sehr breit variieren und liegt üblicherweise im Bereich von 3 Tabletten pro Woche bis hin zu 25 Tabletten pro Tag, bevorzugt 10 Tabletten pro Woche bis 20 Tabletten pro Tag.

Der Weihrauchextrakt wird als Alternative zu Cortison verwendet, um Ödeme im Hirn bei der Behandlung mit der pharmazeutischen Zusammensetzung gemäß der vorliegenden Erfindung zu verhindern. Ödeme treten vermehrt bei der Behandlung von Hirntumoren auf, insbesondere wenn diese schnell zerstört werden, d.h., wenn sie schnell abnehmen.

Die Erfinder der vorliegenden Erfindung haben zudem festgestellt, dass die erfindungsgemäßen Zusammensetzungen bei ihrer Durchgängigkeit ins Gehirn durch Cortisone gestört werden. Überraschenderweise haben die Erfinder jedoch festgestellt, dass als vollwertiger Ersatz gegen die Ödembildung Weihrauch- und/oder Bambusextrakt verwendet werden kann.

Wie bereits oben erwähnt, kommen bevorzugt H15 Präparate als Weihrauchextrakt in Betracht. Als Bambusextrakt, insbesondere der Extrakt von Bambusblättern in Betracht. Es können jedoch auch direkt getrocknete Weihrauchblätter und/oder Bambusblätter in Kapselform in Kombination mit der pharmazeutischen Zusammensetzung gemäß der vorliegenden Erfindung verabreicht werden.

Gemäß einer bevorzugten Weiterbildung dieses Aspektes wird zusätzlich vor dem Vermischen ein Immunmodulator zugegeben, bevorzugt Parapoxvirus ovis, insbesondere bevorzugt vom Stamm D1701.

### Darreichungsformen:

Es zeigt sich, dass die Extrakte oral eingenommen werden können. Die Wirkstoffaufnahme geschieht über den Verdauungstrakt, wobei auch die Blut-Hirn-Schranke von den Wirkstoffen überwunden werden kann. Der ethanolische Auszug ist besonders gut in Honig lösbar, so ist neben der direkten, puren Einnahme des ethanolischen und/oder wässrigen Extraktes auch eine solche gelöst in Honig sinnvoll.

### Behandlungsverlauf anhand eines Patientenbeispiels:

Patient, geboren 1980, diffuses Astrozytom, diagnostiziert im Mai 2016. Kam nach seiner 1. Operation zu Therapie. Erhielt nach der Operation 3 Bestrahlungen und begann parallel mit einer Temozolomid-Chemotherapie. Im Computertomogramm zeigte sich bis März 2018 eine starke Ausbreitung des Tumors diffus und vom Zelltyp maligner werdend. Er begann zunächst mit einer täglichen Dosis von 5 ml eines alkoholischen Extraktes mit der Zusammensetzung gemäß der vorliegenden Erfindung in Honig gelöst vom 2. bis 30. Juni 2016. Seitdem täglich alle 2 Tage 2,5 ml. Das Allgemeinbefinden des Patienten ist gut und im PET ist derzeit kein neues Tumorwachstum mehr zu sehen. Der LSA war nach der Operation 28 und hat sich seit 2020 bei ungefähr 19,8 eingependelt. Der Patient nimmt 2-täglich seit Sommer 2018 5 Tabletten H15.

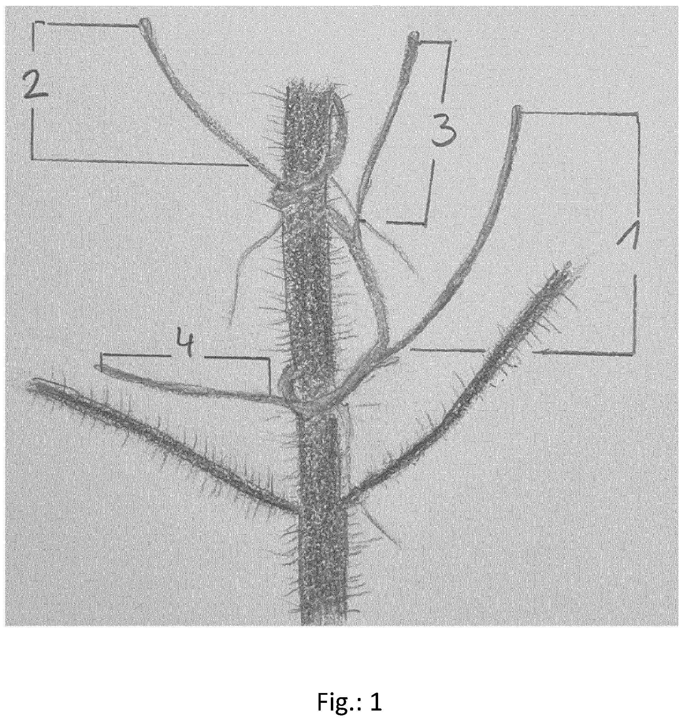

Junge (oder auch sogenannte "frische") Stängel weisen erfindungsgemäß mindestens eine der folgenden Eigenschaften auf:
Junge Stängel die vom Hauptspross /Stängel ausgehen und mindestens 3 bis 4 cm lang sind, bevor sie sich erneut verzweigen (siehe Figur 1). Meist sind diese noch nicht voll ausgefärbt und sehr hellgrün bis gelblich. In der Sonne färben diese sich nach einigen Tagen rötlich, orange und gelb, je nach Wirtspflanzen und Population. Die jungen Stängel sind bei der Ernte etwa 7 Tage alt.

Am besten sollte bei 20 bis 28 Grad bevorzugt bei 23 bis 25 Grad geerntet werden, falls dies wetterbedingt durchführbar ist.

Die Einwaage-Mengen der Cuscuta-Stängel sind in der Tabelle bezüglich der Konzentrationen aufgeführt. Bei 10g Cuscuta-Stängel/100 mL Ethanol erzielt man eine gesättigte Lösung, die am besten zur Einarbeitung in den Honig zu verwenden ist.

Bienenhonig, Bioblütenhonig und Blütenhonig kann im Sinne der vorliegenden Anmeldung als Synonym verwendet werden. Unter Blütenhonig versteht man einen Honig der kein Waldhonig ist und der nicht nur von einer (Haupt)Pflanze gesammelt wird. Also kein (reiner) Akazien- oder Löwenzahn-Honig, sondern aus Nektaren vieler Pflanzenarten bestehend.

Die erfindungsgemäße Zusammensetzung und der erfindungsgemäße Auszug können einen Extrakt aus Weihrauch und/ oder Bambus aufweisen.

Junge Stängelabschnitte 1, 2, 3, 4 sind in der Figur 1 gezeigt; diese sind bevorzugt zu ernten und erfindungsgemäß zu verwenden.

## Patentansprüche

**1.** Auszug zur Bekämpfung von Medulloblastomen und/ oder diffusen Astrozytomen aufweisend Stängel der Cuscuta epithymum, Cuscuta epilinum, Cuscuta europaea, Cuscuta campestris, Cuscuta gronovii und Cuscuta sp.

**2.** Auszug nach Anspruch 1, **dadurch gekennzeichnet, dass** dieser Honig aufweist.

**3.** Auszug nach Anspruch 2, **dadurch gekennzeichnet, dass** der Honig als Biohonig, Bienenhonig, Blütenhonig und/ oder Bioblütenhonig vorliegt.

**4.** Auszug nach mindestens einem der vorhergehenden Ansprüche, **dadurch gekennzeichnet, dass** die Stängel jung sind.

**5.** Auszug nach mindestens einem der vorhergehenden Ansprüche, **dadurch gekennzeichnet, dass** der Auszug mindestens 96% Ethanol und/ oder Aqua ad injectabilia aufweist.

**6.** Auszug nach mindestens einem der vorhergehenden Ansprüche, **dadurch gekennzeichnet, dass** ethanolischer oder wässriger Auszug vorliegt.

**7.** Auszug nach mindestens einem der vorhergehenden Ansprüche, **dadurch gekennzeichnet, dass** der Auszug inaktivierten Parapoxvirus ovis und/ oder Parapoxvirus ovis vom Stamm D1701 aufweist.

**8.** Verfahren zur Herstellung einer therapeutischen Zusammensetzung nach einem der Ansprüche 1 bis 7.

**8.** Verfahren zur Herstellung einer therapeutischen Zusammensetzung nach einem der Ansprüche 1 bis 7, aufweisend:
a) Einbringen einer bestimmten Menge Stängel der Cuscuta epithymum, Cuscuta epilinum, Cuscuta europaea, Cuscuta campestris, Cuscuta gronovii und Cuscuta sp., in eine bestimmte Menge von mindestens 96% Ethanol und/oder Aqua ad injectabilia;
b) Ruhenlassen des Extrakts aus a) von mindestens 15 Stunden;
c) Abfiltrieren der Blätter; und
d) Einarbeiten eines aus Verfahrensschritt c) erhaltenen Wirkextrakts in eine vorbestimmte Menge verflüssigten Honigs.

**9.** Verfahren nach Anspruch 8, **dadurch gekennzeichnet, dass** das Ruhenlassen bei Temperaturen von in etwa 23 bis 26 Grad Celsius erfolgt.

**10.** Verfahren nach Anspruch 8 oder 9, **dadurch gekennzeichnet, dass** das Ruhenlassen zwischen 22 und 30 Stunden andauert.

**11.** Verfahren nach einem der Ansprüche 8 bis 10, **dadurch gekennzeichnet, dass** die vorbestimmte Menge von verflüssigten Honig derart gewählt wird, dass sich beim Einarbeiten des Wirkextrakts in den Honig eine Aufnahmesättigung einstellt.

**12.** Kit of parts zur Behandlung von Hirntumoren wie Medulloblastomen und/oder diffusen Astrozytomen umfassend:
i) eine pharmazeutische Zusammensetzung gemäß einem oder mehreren der Ansprüche 1-8; und
ii) Extrakt aus Weihrauch und/oder Bambus.
